(19) Europäisches Patentamt
European Patent Office
Office européen des brevets

(11) **EP 3 138 501 A1**

(12) **EUROPEAN PATENT APPLICATION**

(43) Date of publication:
08.03.2017 Bulletin 2017/10

(51) Int Cl.:
*A61B 8/08* (2006.01)     *A61B 8/00* (2006.01)
*A61B 5/00* (2006.01)

(21) Application number: 16184894.0

(22) Date of filing: 19.08.2016

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 04.09.2015 JP 2015174389

(71) Applicant: Canon Kabushiki Kaisha
Tokyo 146-8501 (JP)

(72) Inventors:
• SUEHIRA, Nobuhito
Tokyo 146-8501 (JP)
• MASAKI, Fumitaro
Tokyo 146-8501 (JP)
• FUKUTANI, Kazuhiko
Tokyo 146-8501 (JP)
• MIYASATO, Takuro
Tokyo 146-8501 (JP)

(74) Representative: TBK
Bavariaring 4-6
80336 München (DE)

(54) **OBJECT INFORMATION ACQUIRING APPARATUS**

(57) An object information acquiring apparatus comprises a light irradiation unit configured to irradiate an object with light; a sound generating member arranged between the object and the light irradiation unit and configured to generate a substantially planar ultrasonic wave based on the irradiated light; an acoustic wave probe configured to acquire an acoustic wave generated inside the object due to the light and convert the acoustic wave into a first electric signal; and an information generating unit configured to generate information concerning optical characteristics of an interior of the object, based on the first electric signal, wherein the sound generating member allows a part of the light irradiated from the light irradiation unit to pass through the sound generating member to the object.

FIG. 1

EP 3 138 501 A1

**Description**

BACKGROUND OF THE INVENTION

Field of the Invention

The present invention relates to an object information acquiring apparatus that acquires information on the interior of an object.

Description of the Related Art

In recent years, photoacoustic tomography (PAT) has been proposed as a photo-imaging technique.

When a living organism that is an object is irradiated with measurement light such as pulsed laser light, an acoustic wave is generated when the measurement light is absorbed by a living tissue in the object. This phenomenon is referred to as a pohotoacoustic effect, and an acoustic wave resulting from the photoacoustic effect is referred to as a photoacoustic wave. Tissues included in the object absorb light energy at different rates, and thus, resultant photoacoustic waves have different sound pressures. PAT allows information on the optical characteristics of the interior of the object to be imaged by detecting photoacoustic waves using probes and mathematically analyzing reception signals from the probes (this is hereinafter referred to as photoacoustic measurement).

Ultrasonic imaging is known as a method for acquiring structural information on the interior of the object. In ultrasonic imaging, a plurality of ultrasonic probes arranged in a probe transmits ultrasonic waves to the object and receives and analyzes reflected waves generated at interfaces in the object that have different acoustic impedances. Consequently, information (structural information) on the acoustic characteristics on the interior of the object can be imaged (this is hereinafter referred to as ultrasonic measurement).

These techniques have been combined together to develop an apparatus that acquires both information on the optical characteristics of the interior of the object and information on the acoustic characteristics.

For example, with a photoacoustic wave apparatus described in the following document, an ultrasonic image is obtained by irradiating a very small sphere with light, transmitting a resultant photoacoustic wave to the object, and receiving an ultrasonic wave reflected and scattered inside the object.

"Thomas Felix Fehm, Xose Luis Dean-Ben, Daniel Razansky, 'Hybrid optoacoustic and ultrasound imaging in three dimensions and real time by optical excitation of a passive element', Proceedings of SPIE Vo. 9323, 93232X-1"

SUMMARY OF THE INVENTION

When a spherical absorber is utilized to generate an ultrasonic wave as is the case with the apparatus in the above-described document, a spherical wave is generated. However, the spherical wave spreads radially and has its intensity reduced before reaching the object. That is, the related art has room to improve in terms of maintenance of the signal intensity.

The present invention has been developed in view of the above-described problem of the related art.

The present invention provides an object information acquiring apparatus as specified in claims 1 to 8.

The present invention can provide an object information acquiring apparatus that can inexpensively acquire both a photoacoustic image and an ultrasonic image.

Further features of the present invention will become apparent from the following description of exemplary embodiments with reference to the attached drawings.

BRIEF DESCRIPTION OF THE DRAWINGS

FIG. 1 is a diagram describing a configuration of a photoacoustic measurement apparatus according to an embodiment;
FIG. 2 is a diagram describing arrangement of probes in the embodiment;
FIGS. 3A and 3B are diagrams describing the principle of the present invention;
FIGS. 4A and 4B are diagrams describing an arrangement position of a sound generating member in the embodiment; and
FIG. 5 is a process flowchart of a photoacoustic measurement apparatus according to the embodiment.

DESCRIPTION OF THE EMBODIMENTS

An embodiment of the present invention will be described below with reference to the drawings. The same components are in principle denoted by the same reference numerals, and duplicate descriptions are omitted. Numerical values, materials, and the like used in DESCRIPTION OF THE EMBODIMENTS are not intended to limit the scope of the present invention.

In the specification, an acoustic wave generated inside an object or by a light absorber on a surface of the object is referred to as a photoacoustic wave. An electric signal resulting from conversion of a photoacoustic wave is referred to as a photoacoustic wave signal. An image resulting from reconstruction of a photoacoustic wave signal is referred to as a photoacoustic image.

A photoacoustic wave generated by a sound generating member and transmitted to the object is referred to as a measurement ultrasonic wave. A measurement ultrasonic wave reflected and scattered inside the object or by the surface of the object is referred to as a reflected wave. An electric signal resulting from conversion of a reflected wave is referred to as an ultrasonic wave signal. An image resulting from reconstruction of an ultrasonic wave signal is referred to as an ultrasonic image.

An object information acquiring apparatus according to the present embodiment is an apparatus that visualizes, that is, images function information concerning optical characteristics of the interior of the object by irradiating the object with pulsed light and receiving and analyzing a photoacoustic wave generated inside the object as a result of the pulsed light. The information concerning the optical characteristics is substantially an initial sound pressure distribution in the object, a light absorption energy density distribution, an absorption coefficient distribution, or a characteristic distribution concerning the concentration of a material contained in a tissue. Examples of the concentration-related characteristic distribution include the distributions of an oxygen saturation, a value resulting from weighting of the oxygen saturation with an intensity such as absorption coefficient, a total hemoglobin concentration, an oxyhemoglobin concentration, or a deoxyhemoglobin concentration. Further examples of the concentration-related characteristic distribution include the distributions of a glucose concentration, a collagen concentration, a melanin concentration, and the volume fraction of fat or water. The object information acquiring apparatus according to the present embodiment is referred to as a photoacoustic measurement apparatus.

<Problems of the Related Art>

As described above, an apparatus that performs both photoacoustic measurement and ultrasonic measurement utilizes an absorber that allows a spherical wave to be generated, and thus, has difficulty maintaining signal intensity. To solve this problem, a planar absorber needs to be used to generate a planar wave.

However, a planar absorber blocks the object to prevent light from reaching the object, precluding photoacoustic measurement. In other words, when photoacoustic measurement is performed, the planar absorber needs to be moved out of the range of light irradiation, leading to an extended measurement time. Costs of the apparatus are also increased.

The photoacoustic measurement apparatus, which allows these problems to be solved, will be described below.

<System Configuration>

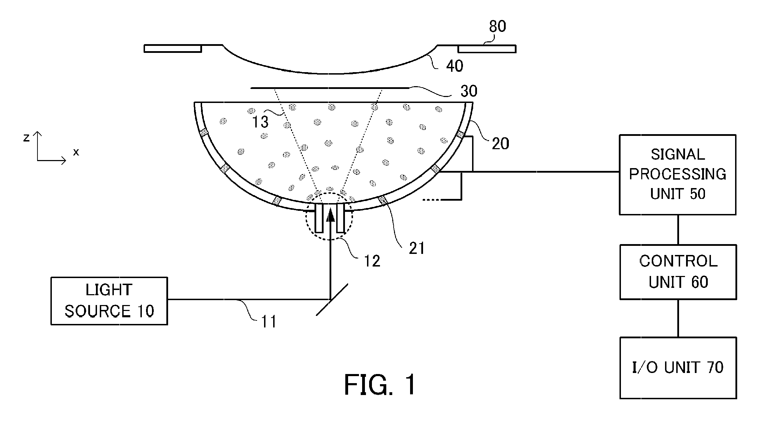

With reference to FIG. 1, a configuration of a photoacoustic measurement apparatus according to a first embodiment will be described. The photoacoustic measurement apparatus according to the present embodiment has a light source 10, a probe unit 20 with a plurality of probes 21, a sound generating member 30, a holding member 40, a signal processing unit 50, a control unit 60, and an I/O unit 70. Units constituting the photoacoustic measurement apparatus according to the present embodiment will be described.

«Light Source 10»

The light source 10 is an apparatus that generates pulsed light radiated to the object. The light source is desirably a laser light source in order to provide high power. However, instead of the laser, a light emitting diode, a flash lamp, or the like may be used. When the laser is used as the light source, any of various lasers may be used such as a solid laser, a gas laser, a dye laser, and a semiconductor laser. Irradiation timing, waveform, intensity, and the like are controlled by a light source control unit not depicted in the drawings. The light source control unit may be integrated with the light source.

The wavelength of the pulsed light is desirably a particular wavelength at which light is absorbed by a particular one of components included in the object and propagates to the interior of the object. Specifically, when the object is a living organism, the wavelength is 700 nm or more and 1100 nm or less.

To allow photoacoustic waves to be effectively generated, light needs to be radiated in a sufficiently short time in accordance with thermal characteristics of the object. When the object is a living organism, pulsed light generated by the light source suitably has a pulse width of approximately 10 nanoseconds to 50 nanoseconds. In the present embodiment, a titanium sapphire laser that is a solid laser is used, and the wavelength is set to 760 nm and 800 nm.

The pulsed light generated by the light source 10 is radiated to the object through a light irradiation portion 12 provided at a bottom of the probe unit 20, described below, via an optical transmission path 11 having optical members such as lenses and mirrors, diffusion plates, and optical fibers (reference numeral 13).

To generate a planar wave with the sound generating member 30, the sound generating member 30 described below is desirably uniformly irradiated with light. For this purpose, the light irradiation portion 12 is provided with a filter and the like to achieve homogeneous light irradiation.

«Probe Unit 20 and Probes 21»

The probes 21 are units for detecting an acoustic wave traveling from the interior of the object and converting the acoustic wave into an electric signal (photoacoustic wave signal). The probe is also referred to as an acoustic-wave probe, an acoustic-wave detector, or a transducer. The acoustic wave in the present invention is typically an ultrasonic wave and includes elastic waves referred to as a sound wave, an ultrasonic wave, a photoacoustic wave, and optical ultrasonic wave.

The photoacoustic wave generated by the living organism is an ultrasonic wave of 100 KHz to 100 MHz, and thus, an ultrasonic detector that can receive the above-described frequency band is used as the probe 21. Specifically, a conversion element utilizing piezoelectric ceramics (PZT), a capacitive micromachined ultrasonic transducer (CMUT), or the like may be used. In the present embodiment, the CMUT is used as the probe. The probe 21 desirably has a high sensitivity and a wide frequency band. The probes 21 utilized in the present embodiment have an aperture of 3 mm per element and has a band of 0.5 to 5 MHz. Providing high sensitivity for low frequency bands allows prevention of blanking of blood vessel images caused by imaging of blood vessel boundaries. A sampling frequency is 50 MHz, and the number of samples is 2048. Output data is signed 12 bits.

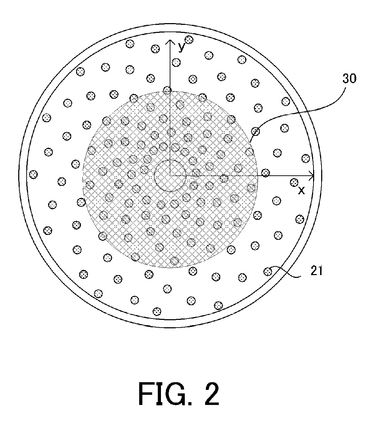

In the present embodiment, a semi-spherical support member as depicted in FIG. 1 (probe unit 20) is provided, and a plurality of the probes 21 is arranged on an inner surface of the probe unit 20. FIG. 2 is a diagram of the photoacoustic measurement apparatus according to the present embodiment as viewed from above in the vertical direction (Z-axis direction). As depicted in FIG. 1, 512 probes 21 are spirally arranged at positions on the probe unit 20 along a semi-spherical surface.

An exit end for pulsed light (light irradiation portion 12) is provided at the bottom of the probe unit 20 and configured to be able to radiate pulsed light to the object in the Z-axis direction (reference numeral 13).

In the present embodiment, the probe unit 20 is configured to be movable along an X-Y plane via an XY stage not depicted in the drawings. This enables irradiation with pulsed light and reception of an acoustic wave to be performed at a plurality of positions with respect to the object, allowing measurement accuracy to be improved.

In the present embodiment, a space between the holding member 40 and the probe unit 20 is filled with a liquid serving as an acoustic matching layer (hereinafter referred to as an acoustic matching liquid, in the present embodiment, water).

«Sound Generating Member 30»

The sound generating member 30 is a member that generates an ultrasonic wave used for ultrasonic measurement (hereinafter referred to as a measurement ultrasonic wave) based on radiated pulsed light. The sound generating member 30 has the property of transmitting a portion of the radiated pulsed light through the sound generating member 30. The ratio between transmission and absorption is set to appropriate values, for example, 50% and 50%, 30% and 70%, or 70% and 30%, according to the amount of light radiated to the living organism and the magnitude of the measurement ultrasonic wave generated. The sound generating member 30 is attached to the probe unit 20 and fixed between the holding member 40 and the probe unit 20. Upon receiving radiated pulsed light, the sound generating member 30 allows a portion of the pulsed light to pass through the sound generating member 30 and generates a measurement ultrasonic wave.

The sound generating member 30 is desirably a thin film formed by containing a light absorbable material in polyethylene terephthalate, polyester, polyethylene, polypropylene, nylon, or vinyl. The light absorbable material may be, for example, a black pigment such as carbon black or a cyan pigment such as copper phthalocyanine. The size of the pigment is, for example, 20 to 80 nm. The mass ratio of the pigment to a resin material is, for example, 0.001 to 1%. Uniform dispersion of the light absorber in the sound generating member 30 enables generation of an ultrasonic wave that is a planar wave. Varying the concentration of the light absorber enables a variation in the ratio between the amount of light transmitted and the measurement ultrasonic wave generated. The characteristics of the light absorber may be varied according to the wavelength of the pulsed light used.

The thickness of the sound generating member 30 is preferably set to 100 μm or less in order to appropriately transmit an acoustic wave (photoacoustic wave or reflected wave) through the sound generating member 30. Varying the thickness of the sound generating member 30 varies the frequency of the measurement ultrasonic wave. For example, an increased thickness of the thin film results in a measurement ultrasonic wave with a low frequency. A reduced thickness of the thin film results in a measurement ultrasonic wave with a high frequency. A plurality of types of sound generating members (for example, sound generating members with different concentrations or thicknesses) may be prepared so that any of the sound generating members that is suitable for the object can be used.

The sound generating member 30 may be produced by applying a pigment ink, a dye ink, or a resin ink to a thin plate such as a polyethylene terephthalate plate, an acrylic plate, or a resin plate. Arranging the ink at intervals like dots allows a substantially planar ultrasonic wave to be generated. Varying the dot intervals of the applied ink allows adjustment of the amount of light transmitted and the intensity of the measurement ultrasonic wave generated. In the present specification, a substantially planar ultrasonic wave is an ultrasonic wave that can be considered as a planar wave at least within the region of the object to be observed.

In the present embodiment, the sound generating member 30 is configured to be movable along an X-Y plane in conjunction with the probe unit 20. The sound generating member 30 may have any shape so long as the sound generating member 30 is large enough to cover an area irradiated with light. The remaining area may be formed of a reflective member so as to prevent light from being transmitted through or absorbed by this area. The sound generating member 30 may be configured to put a lid on an upper portion of the probe unit 20.

The sound generating member 30 may be configured to be immovable. In this case, the sound generating member 30 may be set the same in size as an opening portion of the holding member 40.

Alternatively, the sound generating member 30 may be attached to an arm so as to be switchable or replaceable.

«Holding Member 40»

The holding member 40 is a member shaped like a circular-arc-shaped bowl and installed at a housing (reference numeral 80) of the apparatus. The holding member 40 is a unit for holding the inserted object.

The holding member 40 preferably has a high light transmittance so as to allow transmission of light radiated to the object. The holding member 40 is preferably formed of a material having an acoustic impedance close to the acoustic impedance of the object. Examples of such a material include polyethylene terephthalate, polymethyl pentene, or polyethylene.

<<Signal Processing Unit 50>>

The signal processing unit 50 is a unit including an amplifier and an A/D converter, for amplifying an electric signal resulting from conversion by the probe 21, into a digital signal. The signal resulting from the conversion is transmitted to the control unit 60.

<<Control Unit 60>>

The control unit 60 is a unit for controlling each unit of the photoacoustic measurement apparatus. For example, the control unit 60 performs reception control on the light source, the XY stage, and the probes. The control unit 60 is also an information generating unit for calculating a light amount distribution and reconstructing an image based on an acquired electric signal to acquire information concerning the optical and acoustic characteristics of the interior of the object.

The control unit 60 is typically a workstation having a CPU, a main storage apparatus, and an auxiliary storage apparatus that are independent of one another and executes the above-described process using pre-stored software. The control unit 60 may be hardware designed in a dedicated manner or shared by other units.

The control unit 60 can acquire information input by an apparatus user and present information to the user, via the I/O unit 70 described below. Specifically, based on instructions input by the user, the control unit 60 can perform a change in any of measurement parameters, starting and ending of measurement, selection of a method for processing images, saving of patient information or images, analysis of data, and the like. Finally, an acquired image is output to the I/O unit 70.

<<I/O Unit 70>>

The I/O unit 70 is a unit for accepting an input operation performed by the user and presenting information to the user. The I/O unit 70 is, for example, a liquid crystal display and a keyboard, touch panel display, or the like. The I/O unit 70 is not an essential component of the object information acquiring apparatus according to the present invention.

<Method for Measuring the Object>

Now, a method for measuring the object will be described.

First, the object is irradiated with pulsed light emitted from the light source 10. The pulsed light is radiated to the object through the light irradiation portion 12 via the optical members such as fibers and lenses. The radiated light propagates and diffuses through the object and is absorbed by a substance present in the object. When a portion of the energy of the light having propagated through the object is absorbed by a light absorber such as blood, the light absorber generates an acoustic wave as a result of thermal expansion. If a cancer is present in the living organism, the light is specifically absorbed by the blood in a newborn blood vessel as is the case with the blood in the other normal segments, causing a photoacoustic wave to be generated.

The photoacoustic measurement apparatus according to the present embodiment performs irradiation with pulsed light and reception of a photoacoustic wave with the position of the probe unit 20 displaced. Based on the received photoacoustic wave, information concerning the optical characteristics of the interior of the object (for example, the distributions of an initial sound pressure and an absorption coefficient) is generated.

Besides the photoacoustic wave, a reflected wave reflected and scattered inside the object is received. Then, based on the received reflected wave, information concerning the acoustic characteristics of the interior of the object (for example, information indicative of positions of interfaces with different acoustic impedances) is generated.

<Position of the Sound Generating Member>

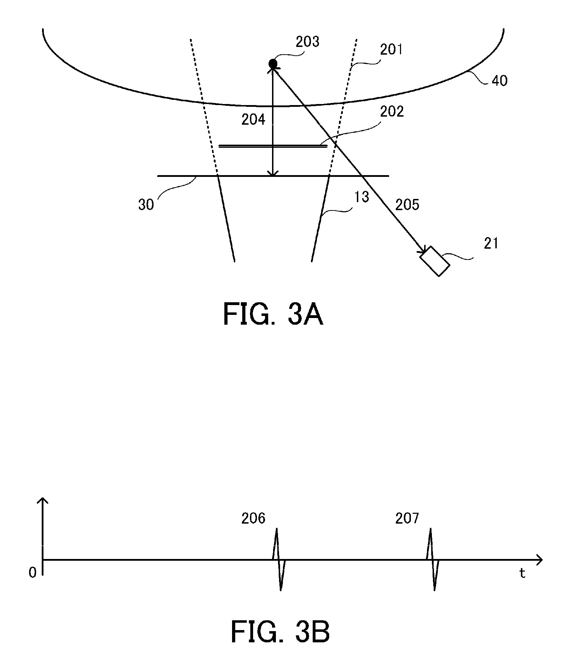

Now, the principle of the present invention will be described with reference to FIG. 3A and FIG. 3B. FIG. 3A is a diagram depicting spatial arrangement of the object and the apparatus. FIG. 3B is a diagram illustrating a temporal variation in acoustic wave received by the probe 21.

Pulsed light 13 from the light irradiation portion is first radiated to the sound generating member 30. The sound generating member 30 transmits a portion of the pulsed light 13 as transmitted light 201, while absorbing a portion of the pulsed light 13 to generate a measurement ultrasonic wave 202 based on the photoacoustic effect.

The transmitted light 201 having been transmitted through the sound generating member 30 is absorbed by a substance 203 present inside the object to generate a photoacoustic wave. For convenience of description, the substance 203 is assumed to be both a light absorber and a sound reflector and scatterer. A photoacoustic wave generated by the substance 203 is received by the probe 21. On the other hand, the measurement ultrasonic wave 202 propagates as a planar wave, and is reflected and scattered by the substance 203 and similarly received by the probe 21.

If the substance 203 exhibits a significant property of being a light absorber, mostly a photoacoustic wave is generated, whereas no measurement ultrasonic wave is generated. An example of such a substance is blood. In contrast, if the substance 203 exhibits a significant property of being a sound reflector and scatterer, no photoacoustic wave is generated. An example of a substance that reflects a measurement ultrasonic wave is a tumor. An example of a substance that scatters a measurement ultrasonic wave is a calcified tissue in the breast.

A photoacoustic wave emitted from the substance 203 is detected as a first signal 206. In FIG. 3B, when a point in time when pulsed light is radiated is assumed to be an origin, the first signal 206 is detected after a time during which the photoacoustic wave propagates a distance from the substance 203 to the probe 21 (reference numeral 205).

A reflected wave is detected as a second signal 207. The second signal 207 is detected after a time during which the measurement ultrasonic wave 202 propagates a distance (reference numeral 204) from the sound generating member 30 to the substance 203 plus the distance (reference numeral 205) from the substance 203 to the probe 21.

A large number of absorbers or scatterers such as the substance 203 are present in the object. For a photoacoustic measurement apparatus that acquires both a photoacoustic wave signal and an ultrasonic wave signal during a single light irradiation operation, these signals, generated inside the object, can desirably be separated from each other. This is particularly because a photoacoustic wave generated by a very small substance is a spherical wave and has an intensity decreasing with increasing distance from the substance to the probe due to attenuation associated with the propagation. Another reason is that overlapping between a photoacoustic wave signal and an ultrasonic wave signal generated away from the probe results in a loud noise.

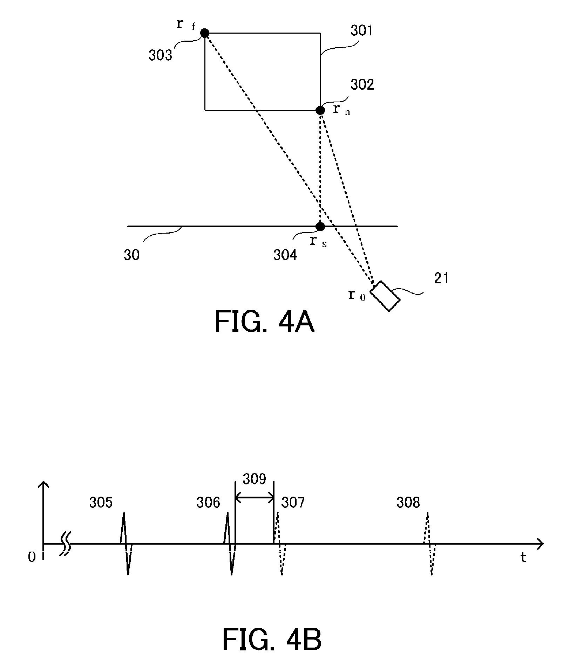

In the present embodiment, the sound generating member 30 is arranged at a position where the sound generating member 30 can separate a photoacoustic wave signal and an ultrasonic wave signal from each other. A specific arrangement position will be described with reference to FIG. 4A and FIG. 4B.

FIG. 4A is a diagram depicting a spatial arrangement in which two substances (substances 302 and 303) are located in an area 301. FIG. 4B is a diagram illustrating signals received by the probe 21. In the present example, the position of the probe 21 is denoted as $r_0$, the position of the substance 302 closest to the position of the probe 21 is denoted as $r_n$, and the position of the farthest substance 303 is denoted as $r_f$. An intersection point between the substance 302 and a perpendicular from the sound generating member 30 is denoted as $r_s$. The area 301 is a predetermined area for which the apparatus acquires information on the interior of the object (a region of interest in the present invention;

hereinafter referred to as a reconstruction area).

The probe 21 detects four signals including a signal 305 corresponding to a photoacoustic wave traveling from the substance 302, a signal 306 corresponding to a reflected wave, a signal 307 traveling from the substance 303, and a signal 308 also corresponding to the reflected wave.

When the reflected wave traveling from the substance 302 can be detected earlier than the photoacoustic wave traveling from the substance 303, the photoacoustic wave signal and the ultrasonic wave signal can be separated from each other. That is, to prevent the two signals from temporally overlapping (a gap 309 can be provided between the signals), the sound generating member 30 needs to be arranged at a position that satisfies a relation represented by Expression (1).

[Math. 1]

$$\left| r_f - r_0 \right| < \left| r_n - r_0 \right| + \left| r_n - r_s \right|$$

$$\mathtt{...\ Expression(1)}$$

The sound generating member 30 is desirably arranged in the reconstruction area at such a position as allows all the probes 21 to satisfy the relation as described above.

If any probe fails to receive the signals due to a relation between the probe 21 and a position where image reconstruction is performed (hereinafter referred to as a reconstruction position), the probe may be excluded. This applies to a case where, for example, the reconstruction position is located outside a solid angle at which signals from the probe 21 can be received. When reconstruction is performed such that signals from probes 21 located closer to the reconstruction position carry more weight, the probes located within an effective range may satisfy Expression (1).

A planar measurement ultrasonic wave is not substantially attenuated while propagating through the acoustic matching liquid. On the other hand, when the sound generating member is a point sound source, a spherical measurement ultrasonic wave is generated. Thus, an increased distance between the sound generating member and the object causes the ultrasonic wave to spread radially to reduce signal intensity. That is, the measurement ultrasonic wave is advantageously a planar wave in order to simultaneously acquire a photoacoustic wave signal and an ultrasonic wave signal.

Separation of the photoacoustic wave signal from the ultrasonic wave signal advantageously enables a reduction in noise attributed to the ultrasonic wave signal and mixed into the photoacoustic wave signal. Moreover, the energy of light and the energy of an ultrasonic wave both applied to the object can be temporally separated from each other. When arranged at the bottom of the probe unit 20, the sound generating member 30 is more suitably prevented from obstructing the sound ray of an acoustic wave received by the probe 21.

<Method for Image Reconstruction>

Now, a process will be described in which an image is reconstructed using the acquired photoacoustic wave signal and ultrasonic wave signal. Image reconstruction is performed by the control unit 60.

First, a process will be described in which information on the optical characteristics of the interior of the object is acquired based on the photoacoustic wave signal. To such a process, a back-projection algorithm in a three-dimensional space can be applied. For example, universal back-projection (UBP) allows an initial sound pressure distribution p (r) to be determined in accordance with Expression (2).

[Math. 2]

$$P(r) = \int_{\Omega_0} b(r_0, t = \left| r - r_0 \right|) \frac{d\Omega_0}{\Omega_0}$$

$$\mathtt{...\ Expression(2)}$$

A term b (ro, t) corresponding to projection data in this case is illustrated in Expression (3). A photoacoustic wave signal detected by a detection element is denoted as $p_d(r_0)$, and the position of each detection element is denoted as $r_0$. Time is denoted as t, and the solid angle of an ultrasonic probe is denoted as $\Omega_0$.

[Math 3]

$$b(r_0, t) = 2 p_d(r_0, t) - 2t \frac{\partial p_d(r_0, t)}{\partial t}$$

$$\mathtt{...\ Expression(3)}$$

A similar back-projection algorithm may be used to reconstruct a reflected wave. However, in view of a time needed for the measurement ultrasonic wave to reach the interior of the object, t is replaced with t' in Expression (4). The position of the sound generating member is denoted as $r_s$, and corresponds to an intersection point between a perpendicular from the reconstruction position r and the sound generating member. Sound velocity is denoted as c. Of course, when the sound velocity varies significantly between the object and the acoustic matching liquid, correction is made with the variation taken into account.

[Math. 4]

$$t' = t - \frac{r - r_s}{c}$$

... Expression(4)

In an ultrasonic apparatus using a linear probe or the like, for example, 128 ultrasonic probes transmits ultrasonic waves to the object and receive the ultrasonic waves reflected inside the object. In such an ultrasonic apparatus, a delay and sum scheme or the like is used to generate images. Such an ultrasonic apparatus can capture a large structure such as a tumor interface but has difficulty capturing small structures such as a calcified tissue. This is because the ultrasonic wave is regularly reflected from a large interface and returns to the ultrasonic probe, whereas the ultrasonic wave is scattered through various agrees by a small structure, with a reduced amount of the ultrasonic wave returning to the ultrasonic probe. However, in the photoacoustic measurement apparatus according to the present embodiment, a large number of the probes 21 are arranged in the probe unit 20 so as to be able to receive signals at various angles, and can thus receive even scattered ultrasonic waves.

<Process Flowchart>

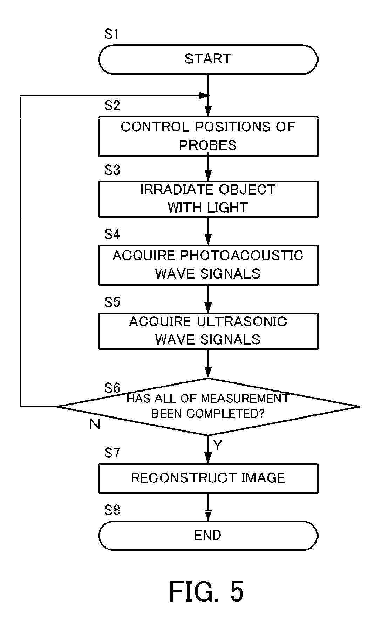

Now, with reference to FIG. 5, a process flowchart will be described which illustrates measurement performed by the photoacoustic measurement apparatus according to the present embodiment (control unit 60).

First, in step S1, measurement is started. In this state, a subject brings the breast into abutting contact with the holding member 40. The space between the holding member 40 and the breast is filled with water, which serves as the acoustic matching liquid, so as to prevent air from being entrapped into the space. When apparatus has gotten ready for measurement, the user performs an operation of starting measurement.

In step S2, the XY stage is controlled to place the probe unit 20 in a desired position. When the XY stage is moved every time pulsed light is radiated, a plurality of light irradiation operations may be performed at the same position before the XY stage is be moved to the next position.

Then, in step S3, the light source 10 is allowed to radiate pulsed light 13. A portion of the pulsed light 13 is transmitted through the sound generating member 30, whereas a portion of the pulsed light 13 is absorbed and changed into the measurement ultrasonic wave. The wavelength of light may be selected according to the contents of the measurement.

Next, in step S4, a photoacoustic wave is received. Transmitted light 201 propagates and diffuses through the breast and is then absorbed by the absorber to generate a photoacoustic wave. The resultant photoacoustic wave is received by the corresponding probe 21.

Then, in step S5, a reflected wave is received. The measurement ultrasonic wave generated by the sound generating member 30 is reflected and scattered inside the breast. Resultant reflected waves are received by the corresponding probes 21. Timing for the reception corresponds to the start of reception of the photoacoustic wave plus a time needed for the measurement ultrasonic wave to reach the object. This is because the photoacoustic wave is generated substantially simultaneously with the irradiation with the pulsed light, whereas the reflected wave needs a longer time corresponding to the amount of time the light travels until the reflection.

In the present example, the data size of each file is reduced through execution of separate steps S4 and S5. However, the photoacoustic wave and the reflected wave may be consecutively acquired, with the resultant data unified. When no ultrasonic measurement is needed, the sound generating member 30 may be removed. In this case, the amount of light radiated to the object may be adjusted as needed.

Step S6 determines whether or not the desired measurement has been completed. When the desired measurement is complete, the process proceeds to step S7. When further measurement is needed, the process returns to step S2 to perform the next measurement.

In step S7, photoacoustic wave signals and ultrasonic wave signals acquired at a plurality of positions are used to generate a photoacoustic image and an ultrasonic image. When a plurality of wavelengths is used, a plurality of photoacoustic signal images is generated. Alternatively, an image may be generated on which calculation of oxygen saturation, etc. has been executed. When a measurement ultrasonic wave is generated with a plurality of wavelengths at the same position, a plurality of ultrasonic images may be obtained. The ultrasonic images may be superimposed into a single ultrasonic image.

In step S8, the process ends. The acquired image is checked, and when the desired image has been acquired, the measurement is ended. Of course, when measurement of the opposite breast or the like is to be performed, the process may return to step S1 to continue the measurement.

As described above, the photoacoustic measurement apparatus according to the present embodiment can acquire both the photoacoustic wave signal and the ultrasonic wave signal during a single light irradiation operation. In particular, when the sound generating member allows a portion of the radiated pulsed light to pass through, the need to move the sound generating member for every measurement is eliminated, enabling a reduction in measurement time. That is, burdens on the subject can be eased. Furthermore, the measurement ends in a short time, enabling suppression of the adverse effect of body motion and facilitating alignment between the photoacoustic image and the ultrasonic image. A function to transmit ultrasonic waves may be omitted from the probes 21, allowing the costs of the apparatus to be kept low.

(Variations)

The description of the embodiments is illustrative for description of the present invention. The present invention may be implemented by changing or combining the embodiments as needed without departing from the spirits of the present invention.

For example, the present invention may be implemented as a photoacoustic measurement apparatus including at least a part of the above-described process. The present invention may be implemented as a method for controlling a photoacoustic measurement apparatus including at least a part of the above-described process. The above-described processes and units may be freely combined together unless the combination is technically contradictory.

Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment (s) , and by a method performed by the computer of the system or apparatus by, for example, reading out and executing the computer executable instructions from the storage medium to perform the functions of one or more of the above-described embodiment (s) and/or controlling the one or more circuits to perform the functions of one or more of the above-described embodiment (s) . The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

An object information acquiring apparatus comprises a light irradiation unit configured to irradiate an object with light; a sound generating member arranged between the object and the light irradiation unit and configured to generate a substantially planar ultrasonic wave based on the irradiated light; an acoustic wave probe configured to acquire an acoustic wave generated inside the object due to the light and convert the acoustic wave into a first electric signal; and an information generating unit configured to generate information concerning optical characteristics of an interior of the object, based on the first electric signal, wherein the sound generating member allows a part of the light irradiated from the light irradiation unit to pass through the sound generating member to the object.

**Claims**

1. An object information acquiring apparatus comprising:

   a light irradiation unit configured to irradiate an object with light;

a sound generating member arranged between the object and the light irradiation unit and configured to generate a substantially planar ultrasonic wave based on the irradiated light;
an acoustic wave probe configured to acquire an acoustic wave generated inside the object due to the light and convert the acoustic wave into a first electric signal; and
an information generating unit configured to generate information concerning optical characteristics of an interior of the object, based on the first electric signal, wherein

the sound generating member allows a part of the light irradiated from the light irradiation unit to pass through the sound generating member to the object.

2. The object information acquiring apparatus according to claim 1, wherein
the acoustic wave probe further acquires the ultrasonic wave reflected inside the object and converts the wave into a second electric signal, and
the information generating unit further generates information concerning acoustic characteristics of the interior of the object, based on the second electric signal.

3. The object information acquiring apparatus according to claim 2, wherein the sound generating member is arranged at a position where an acoustic wave generated in a region of interest in the object due to the light and the ultrasonic wave reflected from the region of interest reach the acoustic wave probe at different points in time.

4. The object information acquiring apparatus according to any one of claims 1 to 3, wherein the sound generating member is a thin film containing a light absorber.

5. The object information acquiring apparatus according to claim 4, wherein the sound generating member is any of polyethylene, polyester, polyethylene terephthalate, polypropylene, nylon, and vinyl.

6. The object information acquiring apparatus according to claim 4 or 5, wherein the light absorber is a pigment.

7. The object information acquiring apparatus according to claim 4 or 5, wherein the light absorber is ink applied at predetermined intervals like dots.

8. The object information acquiring apparatus according to any one of claims 1 to 7, comprising different types of sound generating members that are interchangeable.

FIG. 1

# FIG. 2

FIG. 3A

FIG. 3B

r f
303
301
302
r n

30
304
r s

21
r 0

# FIG. 4A

309
305
306
307
308

0
t

# FIG. 4B

S1

START

S2

CONTROL POSITIONS OF
PROBES

S3

IRRADIATE OBJECT
WITH LIGHT

S4

ACQUIRE PHOTOACOUSTIC
WAVE SIGNALS

S5

ACQUIRE ULTRASONIC
WAVE SIGNALS

S6

HAS ALL OF MEASUREMENT
BEEN COMPLETED?

N

Y

S7

RECONSTRUCT IMAGE

S8

END

FIG. 5

Europäisches
Patentamt

European
Patent Office

Office européen
des brevets

## EUROPEAN SEARCH REPORT

Application Number

EP 16 18 4894

## DOCUMENTS CONSIDERED TO BE RELEVANT

| Category | Citation of document with indication, where appropriate, of relevant passages | Relevant to claim | CLASSIFICATION OF THE APPLICATION  (IPC) |
|---|---|---|---|
| Y | EP 1 493 380 A1 (TOSHIBA KK [JP]) 5 January 2005 (2005-01-05) * abstract * * paragraph [0039] - paragraph [0065] * * paragraph [0113] - paragraph [0153] * * figures 1,2,9-12,15-16 * | 1-8 | INV. A61B8/08 A61B8/00 A61B5/00 |
| Y | GERHILD WURZINGER ET AL: "Simultaneous three-dimensional photoacoustic and laser-ultrasound tomography", BIOMEDICAL OPTICS EXPRESS, vol. 4, no. 8, 1 August 2013 (2013-08-01), pages 1380-1389, XP055329516, United States ISSN: 2156-7085, DOI: 10.1364/BOE.4.001380 * abstract * * page 1381, paragraph 1. Introduction - page 1384, paragraph 1 * * figure 1 * | 1-8 | |
| A | WO 2012/090742 A1 (CANON KK [JP]; FUKUTANI KAZUHIKO [JP]) 5 July 2012 (2012-07-05) * the whole document * | 1-8 | TECHNICAL FIELDS SEARCHED      (IPC) A61B |
| A,D | THOMAS F. FEHM ET AL: "Hybrid optoacoustic and ultrasound imaging in three dimensions and real time by optical excitation of a passive element", OPTICAL SENSING II, vol. 9323, 11 March 2015 (2015-03-11), pages 93232X-1-93232X-6, XP055329110, 1000 20th St. Bellingham WA 98225-6705 USA ISSN: 0277-786X, DOI: 10.1117/12.2080138 ISBN: 978-1-62841-971-9 * pages 93232X-1 - pages 93232X-1 * * abstract * * figure 1 * | 1-8 | |

-/--

The present search report has been drawn up for all claims

| Place of search | Date of completion of the search | Examiner |
|---|---|---|
| Munich | 19 December 2016 | Artikis, T |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

## EUROPEAN SEARCH REPORT

Application Number

EP 16 18 4894

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2013/190595 A1 (ORAEVSKY ALEXANDER A [US] ET AL) 25 July 2013 (2013-07-25)<br>* abstract *<br>* paragraph [0077] - paragraph [0091] *<br>* paragraph [0130] - paragraph [0137] *<br>* figures 3-5,7 *<br>----- | 1-8 | |

TECHNICAL FIELDS
SEARCHED        (IPC)

The present search report has been drawn up for all claims

| Place of search | Date of completion of the search | Examiner |
|---|---|---|
| Munich | 19 December 2016 | Artikis, T |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

 

& : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 16 18 4894

This annex lists the patent family members relating to the patent documents cited in the above-mentioned European search report.
The members are as contained in the European Patent Office EDP file on
The European Patent Office is in no way liable for these particulars which are merely given for the purpose of information.

19-12-2016

| Patent document cited in search report | | Publication date | Patent family member(s) | | Publication date |
|---|---|---|---|---|---|
| EP 1493380 | A1 | 05-01-2005 | CA | 2435990 A1 | 02-01-2005 |
| | | | CN | 1575770 A | 09-02-2005 |
| | | | EP | 1493380 A1 | 05-01-2005 |
| | | | JP | 4406226 B2 | 27-01-2010 |
| | | | JP | 2005021380 A | 27-01-2005 |
| | | | KR | 20050003948 A | 12-01-2005 |
| | | | KR | 20060080562 A | 10-07-2006 |
| | | | US | 2005004458 A1 | 06-01-2005 |
| WO 2012090742 | A1 | 05-07-2012 | JP | 5661451 B2 | 28-01-2015 |
| | | | JP | 2012135462 A | 19-07-2012 |
| | | | US | 2013245420 A1 | 19-09-2013 |
| | | | WO | 2012090742 A1 | 05-07-2012 |
| US 2013190595 | A1 | 25-07-2013 | AU | 2013212213 A1 | 18-09-2014 |
| | | | CA | 2861979 A1 | 01-08-2013 |
| | | | EP | 2806803 A1 | 03-12-2014 |
| | | | JP | 2015507947 A | 16-03-2015 |
| | | | KR | 20140121451 A | 15-10-2014 |
| | | | US | 2013190595 A1 | 25-07-2013 |
| | | | WO | 2013112626 A1 | 01-08-2013 |

EPO FORM P0459

For more details about this annex : see Official Journal of the European Patent Office, No. 12/82